# EUROPEAN PATENT SPECIFICATION

(11) **EP 4 299 725 B1**
(45) Date of publication and mention of the grant of the patent: **03.06.2026**
(21) Application number: 21928186.2
(22) Date of filing: 15.06.2021
(51) Int. Cl.: C12N 7/00, A23K 10/16, A23K 20/195, A01N 63/40, A61K 35/76, A61P 31/04, A61P 31/02, C02F 1/68, C11D 3/38

(54) **NOVEL BACTERIOPHAGE HAVING ENTEROTOXIGENIC ESCHERICHIA COLI-SPECIFIC BACTERICIDAL EFFECT AND ANTIBACTERIAL COMPOSITION COMPRISING SAME**
NEUARTIGER BAKTERIOPHAGE MIT ENTEROTOXIGENER ESCHERICHIA COLI-SPEZIFISCHER BAKTERIZIDER WIRKUNG UND ANTIBAKTERIELLE ZUSAMMENSETZUNG DAMIT
NOUVEAU BACTÉRIOPHAGE AYANT UN EFFET BACTÉRICIDE SPÉCIFIQUE D'ESCHERICHIA COLI ENTÉROTOXIGÈNE ET COMPOSITION ANTIBACTÉRIENNE LE COMPRENANT

(30) Priority: 23.02.2021 KR 20210024235
(43) Date of publication of application: 03.01.2024
(73) Proprietor: CJ Cheiljedang Corporation, Seoul 04560 (KR)
(72) Inventor: JEON, Jong Soo, Seoul 04560 (KR); KIM, Ji Eun, Seoul 04560 (KR); MOON, Jun Ok, Seoul 04560 (KR); CHAE, Jong Pyo, Seoul 04560 (KR); KIM, Yu Jin, Seoul 04560 (KR); LEE, Seung Eun, Seoul 04560 (KR); PARK, Jin Seung, Seoul 04560 (KR)
(74) Representative: Reddie & Grose LLP
(86) International application number: PCT/KR2021/007481
(87) International publication number: WO 2022/181891

(56) References cited:
- WO-A1-2015/156509
- KR-A- 20150 118 836
- KR-A- 20180 068 294
- KR-B1- 101 871 338
- US-A1- 2017 037 380
- US-A1- 2020 190 483
- US-B2- 8 021 657
- BAO H. ET AL: "Isolation and characterization of bacteriophages of Salmonella enterica serovar Pullorum", POULTRY SCIENCE, vol. 90, no. 10, 1 October 2011 (2011-10-01), Oxford, pages 2370 - 2377, XP093358610, ISSN: 0032-5791, Retrieved from the Internet <URL:https://pdf.sciencedirectassets.com/776861/1-s2.0-S0032579119X63439/1-s2.0-S0032579119422931/main.pdf?hash=f33b4112119df00c291a6b5c37e9c62abcc5c1949fdb10dcf90c7cc702cbbb62&host=68042c943591013ac2b2430a89b270f6af2c76d8dfd086a07176afe7c76c2c61&pii=S0032579119422931&tid=spdf-c6408f88-5192-4ae2-906a-325> DOI: 10.3382/ps.2011-01496
- JONCZYK E ET AL: "The influence of external factors on bacteriophages-review", FOLIA MICROBIOLOGICA ; OFFICIAL JOURNAL OF THE INSTITUTE OF MICROBIOLOGY, ACADEMY OF SCIENCES OF THE CZECH REPUBLIC AND CZECHOSLAVAK SOCIETY FOR MICROBIOLOGY, SPRINGER NETHERLANDS, DORDRECHT, vol. 56, no. 3, 31 May 2011 (2011-05-31), pages 191 - 200, XP019921991, ISSN: 1874-9356, DOI: 10.1007/S12223-011-0039-8
- DATABASE NUCLEOTIDE 2 May 2018 (2018-05-02), ANONYMOUS : "Enterobacteria phage vB_EcoM_IME281. complete genome", XP055961143, retrieved from NCBI Database accession no. MH051913

## Description

### [TECHNICAL FIELD]

The present application relates to a bacteriophage having specific bactericidal activity against enterotoxigenic *Escherichia coli* and an antibacterial composition comprising the same.

### [BACKGROUND ART]

*Escherichia coli* (hereinafter, referred to as ' *E.coli')* is a gram-negative short rod bacterium belonging to the genus of *Escherichia,* in the family of *Enterobacteriaceae,* and is one of normal bacterial flora present in the intestinal tract of various animals including mammals. *Escherichia coli* is mostly non-pathogenic and can cause opportunistic infection, but some highly pathogenic strains are known to cause various intestinal diseases and sepsis, and the like in animals including humans.

In particular, according to group breeding of livestock such as hog, poultry, cattle and the like, colibacillosis is emerging as the most common and problematic disease in a livestock farm, and as incidence of colibacillosis increases, it is a situation that causes economically significant losses to a livestock farm, such as growth delay and death and the like due to diarrhea of livestock. In order to prevent and treat colibacillosis, various antibiotics have been used, but as problems of antibiotic abuse of resistance, or problems of possibility of remaining in an animal body have been emerged, many restrictions are set on administration of an antibiotic worldwide at present.

Accordingly, in recent years, since bacteriophages which are bacteria-specific viruses that infect specific bacteria and inhibit growth of bacteria have stronger host specificity than antibiotics, they have been researched as alternatives of new antibiotics, but number thereof is absolutely insufficient so far. Instances thereof can be found e.g. in KR 101 871 338 B1, KR 2018 0068294 A, KR 2015 0118836 or US 2017/037380 A1. Therefore, in order to prevent and treat infectious disease caused by enterotoxigenic *Escherichia coli,* which becomes an important problem in the livestock industry including hog, development of a new bacteriophage, which has high industrial applicability as it has a wide spectrum of bacteriolysis and has excellent acid resistance and heat resistance, has been continuously demanded.

### [Prior art]

### [Patent document]

(Patent document 1) U.S. Patent US 6,942,858 B1

### [DISCLOSURE]

### [TECHNICAL PROBLEM]

An object of the present application is to provide bacteriophage CJ_Eco_20-4 which has specific bactericidal activity against enterotoxigenic *Escherichia coli,* and is deposited under Accession number KCCM12936P, a composition for prevention or treatment of infectious disease caused by enterotoxigenic *Escherichia coli,* an antibiotic, a feed additive, a drinking water additive, a disinfectant, or a detergent comprising the same as an active ingredient, or a feed comprising the feed additive, or drinking water comprising the drinking water additive.

Another object of the present invention is to provide a non therapeutic method for prevention or treatment of infectious disease caused by enterotoxigenic *Escherichia coli* using the bacteriophage CJ_Eco_20-4 or the composition for prevention or treatment of infectious disease caused by enterotoxigenic *Escherichia coli* comprising the same as an active ingredient.

Other object of the present invention is to provide a use for preparation of a prophylactic agent or a therapeutic agent of infectious disease caused by enterotoxigenic *Escherichia coli* of the bacteriophage CJ_Eco_20-4 or the composition for prevention or treatment of infectious disease caused by enterotoxigenic *Escherichia coli* comprising the same as an active ingredient.

### [TECHNICAL SOLUTION]

One aspect provides bacteriophage CJ_Eco_20-4 which has specific bactericidal activity against enterotoxigenic Escherichia coli (ETEC), and is deposited under Accession number KCCM12936P.

The term used herein, "Enterotoxigenic *Escherichia coli* (ETEC)"is one of pathogenic Escherichia coli, and corresponds to a causative bacterium that causes infectious disease in an animal such as hog, and the like.

The term used herein, "bacteriophage" is a bacteria-specific virus which infects a specific bacterium and inhibits growth of the corresponding bacterium, and means a virus comprising single or double-stranded DNA or RNA as a genetic material.

Another aspect provides a composition for prevention or treatment of infectious disease caused by enterotoxigenic *Escherichia coli* comprising the bacteriophage CJ_Eco_20-4 as an active ingredient.

The bacteriophage CJ_Eco_20-4 is as described above.

The bacteriophage CJ_Eco_20-4 has specific bactericidal activity against enterotoxigenic *Escherichia coli,* so it can prevent infectious disease caused by enterotoxigenic *Escherichia coli* by killing enterotoxigenic *Escherichia coli* entering the subject, and inhibiting infection thereby, when administered before occurrence of infectious disease caused by enterotoxigenic *Escherichia coli* into a subject. In addition, when the bacteriophage CJ_Eco_20-4 is administered into a subject in which infectious disease caused by enterotoxigenic *Escherichia coli* is developed, by killing enterotoxigenic *Escherichia coli* present in the subject, it can alleviate or extinct symptoms shown thereby, and thus, it can treat infectious disease caused by enterotoxigenic *Escherichia coli.*

The term used herein, "prevention" may mean all behaviors which inhibit or delay occurrence of disease in a subject by administration of a bacteriophage or a composition, and the term, "treatment" may mean all behaviors which improve, alleviate, or extinct symptoms of disease in a subject by administration of a bacteriophage or a composition.

The infectious disease caused by enterotoxigenic *Escherichia coli* may be for example, colibacillosis, but not limited thereto.

The term used herein, "colibacillosis" means a disease occurring as a pathogenic E. coli is infected into livestock, and as symptoms, septicemia, diarrhea (neonatal diarrhea and postweaning diarrhea), toxemia (edema disease and cerebrospinal angiosis) and the like may appear. Among them, the septicemia is an acute systemic infectious disease frequently occurring during the neonatal period within 2~3 days after birth and has a high mortality rate. The diarrhea is an intestinal infectious disease that frequently occurs during the lactation period and immediately after weaning within 1~2 weeks of age, and causes death or developmental disorders. The toxemia occurs mainly in piglets and the like of 8-12 weeks after weaning, and swells the whole body, shows neurological symptoms, and often leads to sudden death.

The composition for prevention or treatment of infectious disease caused by enterotoxigenic E. coli may comprise the bacteriophage CJ_Eco_20-4 at 1 X 10 ² to 1 X 10 ²⁰ PFU/mL or 1 X 10 ² to 1 X 10 ²⁰ PFU/g, and for example, it may comprise the bacteriophage CJ_Eco_20-4 at 1 X 10 ² to 1 X 10 ¹² PFU/mL or 1 X 10 ⁵ to 1 X 10 ¹⁰ PFU/g.

The composition for prevention or treatment of infectious disease caused by enterotoxigenic E. coli may further comprise a pharmaceutically acceptable carrier, and it may be provided as a food, medicine, feed additive or drinking water additive or the like as formulated with the carrier.

The term used herein, "pharmaceutically acceptable carrier" may mean a carrier or diluent which does not stimulate an organism and does not inhibit biological activity and characteristics of an administered compound.

The kind of the carrier to be comprised in the composition is not particularly limited, and any pharmaceutically acceptable carrier commonly used in the art may be used. Non-restrictive examples of the carrier may include saline solution, sterilized water, Ringer's solution, buffered saline solution, albumin injection solution, dextrose solution, malto-dextrin solution, glycerol and the like. They may be used alone or two or more kinds may be mixed and used, and if needed, it may be formulated as a formulation for injection such as solution, suspension, emulsion and the like, pill, capsule, granule or tablet by additionally adding a diluent, a dispersing agent, a binder and a lubricant.

The composition for prevention or treatment of infectious disease caused by enterotoxigenic E. coli may be administered by oral administration or parenteral administration, and a method for applying or spraying to a disease area may be used. In case of parenteral administration, it may be administered using intravenous administration, intraperitoneal administration, intramuscular administration, subcutaneous administration or topical administration. An amount of applying, spraying and administering of the composition varies according to factors such as formulation method, administration method, target animal and patient's age, body weight, gender, degree of disease symptoms, food, administration time, administration route, excretion rate and reaction sensitivity, and a commonly skilled doctor or veterinarian may easily determine and prescribe an effective dose for desired treatment.

As a formulation for oral administration of the composition for prevention or treatment of infectious disease caused by enterotoxigenic E. coli, for example, it may be formulated as a tablet, troche, lozenge, aqueous or oil suspension, prepared powder or granule, emulsion, hard or soft capsule, syrup or elixir. In order to formulate it as a formulation such as a tablet or capsule or the like, it may comprise a binder such as lactose, saccharose, sorbitol, mannitol, starch, amylopectin, cellulose or gelatin, an excipient such as dicalcium phosphate, a disintegrant such as corn starch or sweat potato starch, or a lubricant such as magnesium stearate, calcium stearate, sodium stearyl fumarate, or polyethylene glycol wax, and in case of the capsule formulation, a liquid carrier such as fat oil may be further contained in addition to the afore-mentioned materials.

As a formulation for parenteral administration of the composition for prevention or treatment of infectious disease caused by enterotoxigenic E. coli, it may be formulated in a form for injection such as subcutaneous injection, intravenous injection or intramuscular injection or the like, or for spray such as aerosol allowing inhalation through a suppository injection method or a respirator. In order to formulate it in a formulation for injection, the composition of the present invention may be prepared as a solution or suspension by mixing with a stabilizer or buffer in water, and this may be formulated for unit administration of an ample or vial. When it is formulated as a spray such as aerosol and the like, a propellant or the like may be blended with an additive so that a water-dispersed concentrate or wet powder.

The composition for prevention or treatment of infectious disease caused by enterotoxigenic E. coli may comprise a preservative, stabilizer, wetting agent or emulsifier, cryoprotectant, or excipient, or the like.

The preservative, stabilizer, or excipient may be comprised in an effective dose sufficient for reducing deterioration of the composition in the composition.

The term used herein, "deterioration" may comprise a decrease in the number of bacteria, a decrease in activity, of the bacteriophage CJ_Eco_20-4 comprised in a composition and the like, or a combination thereof.

For example, as the composition comprises an effective dose of preservative, stabilizer or excipient, sufficient for reducing deterioration of the composition, differently from a bacteriophage present in nature, the bacteriophage CJ_Eco_20-4 may survive or maintain activity for a longer period of time in the composition.

As the preservative, stabilizer, or excipient, those commonly used in the art may be used without limitation, as long as they can reduce deterioration of the composition. For example, the stabilizer may be any one or more selected from the group consisting of alginate, sodium alginate, casein, sodium casein, cellulose, methyl cellulose, carboxymethylcellulose, sodium carboxymethylcellulose, pectin, gelatin, Arabic gum, xanthan gum, dextran, cyclodextrin and starch.

The cryoprotectant may be comprised in an effective dose sufficient for reducing deterioration of the composition in the composition, when the composition is freeze-dried. For example, differently from a bacteriophage present in nature which has lowered activity or cannot survive in the freeze-dried composition, the bacteriophage activity may be maintained or the bacteriophage may survive even in the freeze-dried composition, as the composition comprises an effective dose of cryoprotectant sufficient for reducing deterioration of the composition in a freeze-dried state.

As the cryoprotectant, those commonly used in the art may be used without limitation, as long as it is to reduce deterioration of the composition in a freeze-dried state. For example, the cryoprotectant may be any one or more selected from the group consisting of glycerol, ethylene glycol, propylene glycol, dimethyl sulfoxide (DMSO), glucose, trehalose, maltodextrin, dextrin, skim milk and starch.

Other aspect provides an antibiotic comprising the bacteriophage CJ_Eco_20-4 as an active ingredient.

The bacteriophage CJ_Eco_20-4 is as described above.

The term used herein, "antibiotic" may mean an agent capable of killing bacteria or inhibiting growth of bacteria, and may collectively call preservatives, bactericides, and antimicrobials.

The antibiotic comprising the bacteriophage CJ_Eco_20-4 as an active ingredient has very high specificity against enterotoxigenic E. coli compared to conventional antibiotics, so it cannot kill beneficial bacteria and can kill specific pathogenic bacteria only, and it does not induce drug resistance, so it can exhibit an effect of extending a product lifespan compared to conventional antibiotics.

The antibiotic may be prepared by a method for preparation commonly used in the art.

The antibiotic may comprise an effective dose of additive sufficient for reducing deterioration of the antibiotic, and the additive may include a preservative, stabilizer, excipient, or cryoprotectant, or the like. As the antibiotic comprises the additive, differently from a bacteriophage present in nature, the bacteriophage CJ_Eco_20-4 may survive or maintain activity for a longer period of time in the antibiotic. As the preservative, stabilizer, excipient or cryoprotectant, those commonly used in the art may be used without limitation, as long as it can reduce deterioration of the antibiotic.

Other aspect provides a feed additive comprising the bacteriophage CJ_Eco_20-4 as an active ingredient. Other aspect provides a feed comprising the feed additive. Other aspect provides a drinking water additive comprising the bacteriophage CJ_Eco_20-4 as an active ingredient. Other aspect provides drinking water comprising the drinking water additive.

The bacteriophage CJ_Eco_20-4 is as described above.

The feed additive or drinking water additive may be used by a method of mixing in a feed or drinking water as prepared in a form of a composition comprising the bacteriophage CJ_Eco_20-4, or directly adding the bacteriophage CJ_Eco_20-4 during preparation of the feed or drinking water.

The bacteriophage CJ_Eco_20-4 comprised in the feed additive or drinking water additive may be in a liquid state or in a dried solid state, and specifically, it may be in a dried powder form. The method of drying of the bacteriophage CJ_Eco_20-4 may be air drying, natural drying, spray drying or freeze-drying, but not limited thereto. The bacteriophage CJ_Eco_20-4 may be mixed in a dried powder form in an amount of 0.05 to 10 % by weight, specifically, 0.1 to 2 % by weight of the weight of the feed additive or drinking water additive.

The feed additive or drinking water additive comprising the bacteriophage CJ_Eco_20-4 as an active ingredient may further comprise other additives if needed. Non-restrictive examples of the usable additives include a binder, emulsifier, preservative and the like added to prevent deterioration of a feed or drinking water; and an amino acid agent, vitamin agent, enzyme agent, probiotic, flavoring agent, non-protein nitrogen compound (NPN), silicate agent, buffer, coloring agent, extractant or oligosaccharide or the like, and they may be added along or two or more kinds may be added together.

Raw materials other than the bacteriophage CJ_Eco_20-4 comprised in the feed or drinking water may be used without limitation as those commonly used in the art. Non-restrictive examples of the feed may include vegetable feeds such as grains, root fruits, food processing by-products, algae, fibers, pharmaceutical by-products, oils and fats, starches, meal or grain by-products, or the like; and animal feeds such as proteins, inorganic materials, oils and fats, minerals, oils and fats, monocyte proteins, zooplanktons or food, or the like. They may be used alone or used by mixing 2 or more kinds.

The feed additive may be comprised in an amount of 0.05 to 10 parts by weight, for example, 0.1 to 2 parts by weight, based on 100 parts by weight of feed. The drinking water additive may be comprised in an amount of 0.0001 to 0.01 parts by weight, for example, 0.001 to 0.005 parts by weight, based on 100 parts by weight of drinking water.

The feed additive, feed, drinking water additive or drinking water may be prepared by a method for preparation commonly used in the art.

The feed additive, feed, drinking water additive or drinking water may comprise an effective dose of an additive sufficient to reduce deterioration thereof, and the additive may be a preservative, stabilizer, excipient or cryoprotectant, or the like. The feed additive, feed, drinking water additive or drinking water may survive or maintain activity for a longer period than the bacteriophage CJ_Eco_20-4 in the feed additive, feed, drinking water additive or drinking water, differently from the bacteriophage present in nature, by comprising the additive. As long as the preservative, stabilizer, excipient, or cryoprotectant can reduce deterioration of the feed additive, feed, drinking water additive or drinking water, those commonly used in the art may be used without limitation.

Other aspect provides a disinfectant comprising the bacteriophage CJ_Eco_20-4 as an active ingredient. Other aspect provides a detergent comprising the bacteriophage CJ_Eco_20-4 as an active ingredient.

The bacteriophage CJ_Eco_20-4 is as described above.

The formulation of the disinfectant or detergent is not particularly limited, and it may be prepared into a formulation commonly known in the art and used. The disinfectant or detergent may be prepared by a method for preparation commonly used in the art.

The disinfectant may be sprayed to remove enterotoxigenic E. coli, and may be sprayed on animal activity areas, slaughterhouses, dead areas, cooking places or cooking facilities or the like, but not limited thereto.

The detergent may be used for a use of washing a skin surface or each body part or the like of an animal which is exposed to or likely to be exposed to enterotoxigenic E. coli, but not limited thereto.

The disinfectant or detergent may comprise an effective dose of an additive sufficient to reduce deterioration thereof, and the additive may be a preservative, stabilizer, excipient, or cryoprotectant, or the like. The disinfectant or detergent may survive or maintain activity for a longer period than the bacteriophage CJ_Eco_20-4 in the disinfectant or detergent, differently from the bacteriophage present in nature, by comprising the additive. As long as the preservative, stabilizer, excipient, or cryoprotectant can reduce deterioration of the disinfectant or detergent, those commonly used in the art may be used without limitation.

Other aspect provides a non therapeutic method for prevention or treatment of infectious disease caused by enterotoxigenic Escherichia coli, comprising administering the bacteriophage CJ_Eco_20-4, or a composition for prevention or treatment of infectious disease caused by enterotoxigenic E. coli comprising this as an active ingredient into an animal except for humans.

The bacteriophage CJ_Eco_20-4, composition for prevention or treatment of infectious disease caused by enterotoxigenic E. coli comprising this as an active ingredient, and infectious disease caused by enterotoxigenic E. coli are as described above. Specifically, the infectious disease caused by enterotoxigenic E. coli may be for example, colibacillosis.

The non therapeutic method for prevention or treatment specifically comprises administering a pharmaceutically effective dose of the bacteriophage CJ_Eco_20-4, or a composition comprising this as an active ingredient into an animal except for humans, which are infected or at risk of being infected with enterotoxigenic E. coli. The bacteriophage CJ_Eco_20-4, or a composition comprising this as an active ingredient may be administered into an animal in a form of pharmaceutical formulations, or may be administered by a method for mixing in a form of feed additives or drinking water additives into a feed or drinking water of an animal and feeding this.

The administration route of the bacteriophage CJ_Eco_20-4 or composition comprising the same as an active ingredient may be various oral or parenteral routes as long as it can reach target tissue, and specifically, it may be administered by a common method through oral, rectal, local, intravenous, intraperitoneal, intramuscular, intraarterial, transdermal, intranasal, inhalation and the like.

A suitable daily total amount used of the bacteriophage CJ_Eco_20-4 or composition comprising the same as an active ingredient may be determined within the scope of correct medical judgment by a treatment doctor, and this is an obvious matter to those skilled in the art.

A specific pharmaceutically effective dose of the bacteriophage CJ_Eco_20-4 or composition comprising the same as an active ingredient for a specific animal, may be determined in consideration of the type and degree of a response to be achieved, the age, body weight, general health condition, gender or diet of the corresponding subject, as well as, the administration time and administration route of the bacteriophage CJ_Eco_20-4 or the composition comprising the same as an active ingredient, and the secretion rate of the composition, treatment period and the like, and it may vary depending on various factors including drugs and other components of the composition used simultaneously or at different times together and similar factors well known in the medical field.

As the animal except for humans, any animal which can be infected by enterotoxigenic *Escherichia coli* is not particularly limited. For example, the animal may be a bird or mammal, and specifically, it may include chickens, ducks, pigs, and the like, but not limited thereto.

Other aspect provides a use for preparation of a prophylactic agent or a therapeutic agent of infectious disease caused by enterotoxigenic *Escherichia coli* of a composition for prevention or treatment of infectious disease caused by enterotoxigenic *Escherichia coli* of the bacteriophage CJ_Eco_20-4, or a composition for prevention or treatment of infectious disease caused by enterotoxigenic *Escherichia coli* comprising the same as an active ingredient.

The bacteriophage CJ_Eco_20-4, composition for prevention or treatment of infectious disease caused by enterotoxigenic *Escherichia coli* comprising the same as an active ingredient, and infectious disease caused by enterotoxigenic *Escherichia coli* are as described above. Specifically, the infectious disease caused by enterotoxigenic *Escherichia coli* may be for example, colibacillosis.

### [ADVANTAGEOUS EFFECTS]

The bacteriophage CJ_Eco_20-4 has an effect of specifically killing enterotoxigenic *Escherichia coli,* and has excellent acid resistance and heat resistance, so it can be widely utilized for an antibiotic, a feed additive, a drinking water additive, a feed, drinking water, a disinfectant or a detergent, or the like as a use for prevention or treatment of infectious disease caused by enterotoxigenic *Escherichia coli.*

### [BRIEF DESCRIPTION OF THE DRAWINGS]

FIG. 1 is an electron microscope photograph of the bacteriophage CJ_Eco_20-4.
FIG. 2 is a result graph of confirming pH stability of the bacteriophage CJ_Eco_20-4.
FIG. 3 is a result graph of confirming stability at 60°C of the bacteriophage CJ_Eco_20-4.
FIG. 4 is a result graph of confirming lytic activity against an enterotoxigenic *Escherichia coli* (ETEC) strain of the bacteriophage CJ_Eco_20-4.
FIG. 5 is a result graph of confirming lytic activity against an enterotoxigenic *Escherichia coli* (ETEC) strain of the bacteriophage CJ_Eco_20-4

### [MODE FOR INVENTION]

Hereinafter, the present invention will be described in more detail by examples.

### Example 1. Separation of bacteriophage having bactericidal activity against enterotoxigenic Escherichia coli (ETEC)

### Example 1-1. Preparation of fecal sample pretreatment solution

Fecal samples of hog, poultry and cattle were collected from farms in Seoul, Gyeonggi-do, Chungcheong-do and Gyeongsang-do areas of Korea, and 20g of each sample was diluted in PBS of 80 mL and centrifuged at 10,000 rpm for 15 minutes. The supernatant was filtered with a 0.2 µm filter, and then 10%(w/v) sodium chloride aqueous solution was added to the filtrate, and stored at 4°C for 12 hours. After that, 10%(w/v) polyethylene glycol 8000 (Sigma-Aldrich, Cat. No. P2139) was added thereto, and stored at 4°C for 12 hours, and then for concentration, it was centrifuged at 15,000 rpm for 1 hour to remove the supernatant. The precipitate was dissolved in 10 mL of SM buffer (5.8 g/L sodium chloride, 2 g/L MgSO ₄·7H ₂O, 0.05M Tris-Cl (pH 7.5)), and then filtered with a 0.2 µm filter, and the filtrate was stored at 4°C.

### Example 1-2. Production of bacteriophage concentrated solution

ETEC strains separated from fecal samples collected from livestock farms of Seoul, Gyeonggi-do, Chungcheong-do and Gyeongsang-do areas of Korea and ETEC strains sold from National Veterinary Research and Quarantine Service were inoculated into an LB medium (Luria-Bertani medium; 10 g/L tryptone, 5 g/L yeast extract, 10 g/L sodium chloride) of 2mL, and cultured at 30°C and 180 rpm for 20 hours, and then, the ETEC strain cultured solution of 1mL and the fecal sample pretreatment solution obtained from Example 1-1 of 1 mL were inoculated into the LB medium of 50 mL, and mixed and cultured at 30°C and 180 rpm for 20 hours. After the mixed cultured solution was centrifuged at 6,000 rpm for 20 minutes, the supernatant was filtered with a 0.2 µm filter, and 10%(w/v) polyethylene glycol 8000 was added thereto, and stored at 4°C for 12 hours. After that, for concentration, it was centrifuged at 15,000 rpm for 1 hour to remove the supernatant, and the precipitate was dissolved in 1mL of SM buffer, and it was filtered with a 0.2 µm filter, and the filtrate was stored at 4°C.

### Example 1-3. Screening and separation of bacteriophage

50 µL of the bacteriophage concentrated solution produced in Example 1-2 was mixed with 5 mL of 0.7%(w/v) agar (BD DIFCO, Cat. No. 44164) and 50 µL of the cultured solution in which the ETEC strain as same as used in Example 1-2 was cultured with shaking so that the absorbance (O.D.) was 2, and using a plate medium with a diameter of 150 mm, double-layer agar plaque assay was performed. Plaques formed on soft agar were punched with a 200 µL tip, and added to 0.5 mL of SM buffer to elute. Double-layer agar plaque assay was repeated for a solution comprising the eluted bacteriophage until a single plaque in the same form was formed to separate a solution comprising a pure bacteriophage. The obtained solution comprising the bacteriophage was filtered with a 0.2 µm filter, and 10%(w/v) polyethylene glycol 8000 was added thereto, and stored at 4°C for 12 hours. After that, for concentration, it was centrifuged at 15,000 rpm for 1 hour to remove the supernatant, and the precipitate was dissolved in 1 mL of SM buffer, and it was filtered with a 0.2 µm filter, and the filtrate was stored at 4°C.

### Example 2. Whole genome sequencing (WGS) of separated bacteriophage

DNA was extracted from 1 mL of the pure separated bacteriophage concentrated solution in Example 1-3 using CsCl gradient method and a phage DNA separation kit (Norgen Biotek-Corp. Kit, Cat. No. 46800). Whole genome sequencing was performed by requesting to Macrogen Inc., and genes were combined using De novo assembly software (SPAdes 3.13.0), and Open reading frame (ORF) was performed using GeneMark.hmm and NCBI ORF finder. The function of each ORF was annotated using BLASTP (E values of <0.1) and PSI-BLAST (E value of <0.005) programs.

As a result, it was confirmed that the separated bacteriophage had the base sequence of SEQ ID NO: 1 with 170,632 bp, 276 ORF, G+C content 39.6%, and this showed the sequence identity of 92% with conventionally reported *Enterobacteria* phage vB_EcoM_IME281 (MH051913.1), but it was confirmed that there was no bacteriophage of which all fragments matched 100%, and it could be seen that the bacteriophage was a separated bacteriophage. Accordingly, this bacteriophage was named bacteriophage CJ_Eco_20-4, and deposited to Korean Culture Center of Microorganisms, which is an international depository institution under Budapest Treaty on January 18, 2021, and was given Accession number KCCM12936P.

### Example 3. Morphology analysis of bacteriophage CJ_Eco_20-4

In order to obtain a high purity of bacteriophage solution, CsCl gradient method was performed. Specifically, a CsCl solution dissolved in SM buffer of which density was 1.7, 1.5, 1.45 or 1.3 was prepared, and the CsCl solution was aliquoted in a 15 mL ultracentrifuge tube (Beckman Coulter, Cat. No. Z00901SCA) by 2 mL each so as to layer from high density to low density, and 2 mL of the bacteriophage CJ_Eco_20-4 concentrated solution obtained in Example 1-3 was aliquoted at the top. This was centrifuged at 4°C and 25,000 rpm for 2 hours, and then only the white bacteriophage layer formed in the tube was collected with a syringe (Satorius, Cat. No. 17822-K). After dropping 1 µL of the collected bacteriophage solution on carbon-coated copper grid, it was stained with 2% uranyl acetate for 15 seconds and the morphology was observed with an electron microscope (TEM, JEOL JEM-101, Tokyo, Japan).

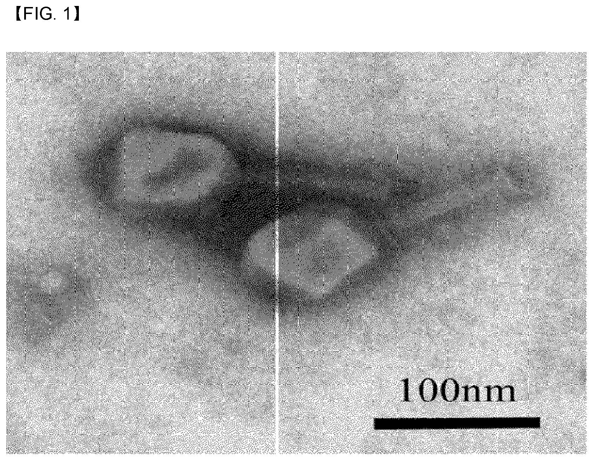

As a result, as shown in FIG. 1, it was observed that the bacteriophage CJ_Eco_20-4 had a morphologically icosahedral head and a contractile tail with a length of about 100 nm, and thereby, it could be seen that it belonged to *Caudovirales* order, *Myoviridae* family.

### Example 4. Evaluation of pH stability of bacteriophage CJ_Eco_20-4

In order to confirm that the bacteriophage CJ_Eco_20-4 has stability in a broad pH range, a solution with pH 3, 4, 7, 7.5 or 10 (pH 3, 4: 0.2M sodium acetate solution; pH 7, 7.5: 0.2M sodium phosphate solution; and pH 10: 0.2M Tris(Tris-HCl) solution) was prepared. After 450 µL of the solution by each pH and 50 µL of the bacteriophage solution of 2 X 10 ¹⁰ PFU/mL were mixed and stood at 4°C for 2 hours, double-layer agar plaque assay was performed to evaluate titer increase and decrease.

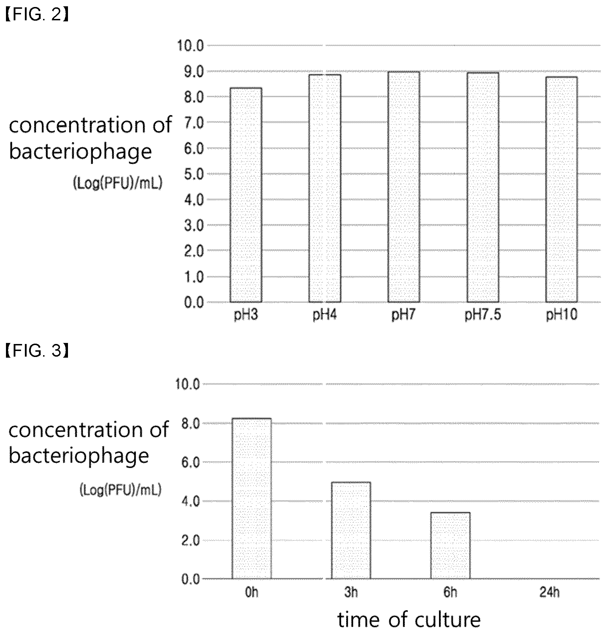

As a result, as shown in FIG. 2, the bacteriophage CJ_Eco_20-4 was stable without losing activity in a range of pH 3 to 10, so it could be seen that it was a bacteriophage having excellent acid resistance.

### Example 5. Evaluation of thermal stability of bacteriophage CJ_Eco_20-4

In order to confirm whether the bacteriophage CJ_Eco_20-4 had stability at a high temperature, after standing 500 µL of the bacteriophage solution of 2 X 10 ⁸ PFU/mL at 60°C for 0, 3, 6 or 24 hours, double-layer agar plaque assay was performed to evaluate titer increase and decrease.

As a result, as shown in FIG. 3, the bacteriophage CJ_Eco_20-4 showed a decrease in activity of about 3.3 logs compared to the control group exposed for 0 hour, when it is exposed at 60°C for 3 hours, and showed a decrease in activity of about 4.8 logs compared to the control group when exposed for 6 hours. Therefore, it could be seen that the bacteriophage CJ_Eco_20-4 was a bacteriophage having excellent heat resistance.

### Example 6. Evaluation of bacteriolysis spectrum of bacteriophage CJ_Eco_20-4

In order to evaluate the bacteriolysis range of the bacteriophage CJ_Eco_20-4, a total of 10 kinds of the ETEC strain from fecal samples collected from livestock farms of Seoul, Gyeonggi-do, Chungcheong-do and Gyeongsang-do areas of Korea and ETEC strain sold from National Veterinary Research and Quarantine Service were cultured in an LB liquid medium, respectively, and then 50 µL of each strain culture was inoculated into 5 mL of 0.7% soft agar and poured in a petri dish and plated, and then stood for 5 minutes. After that, 10 µL of the bacteriophage CJ_Eco_20-4 concentrated solution obtained in Example 1-3 was spotted on soft agar, and then standing cultured at 30°C for 20 hours. After completing the culture, the bacteriolysis range of the bacteriophage CJ_Eco_20-4 was evaluated according to presence or absence of plaques formed on soft agar.

**[Table 1]**

| ETEC strain name | Presence or absence of plaque formation | ETEC strain name | Presence or absence of plaque formation |
|---|---|---|---|
| *E.coli* SNU273 | + | Philippine T5 | + |
| *E.coli* SNU287 | + | F18-31 | + |
| *E.coli* SNU337 | + | ETEC-3 | + |
| 12-194-1 | + | ETEC-4 | + |
| 12-364-1 | + | ETEC-11 | + |

| | | | |
|---|---|---|---|
| (+: Plaques are formed) | | | |

As a result, as shown in Table 1, it was confirmed that plaques were formed in all the 10 kinds of the tested ETEC strains, so the bacteriophage CJ_Eco_20-4 had bactericidal activity against widespread ETEC strains.

### Example 7. Evaluation of bacteriolytic activity of bacteriophage CJ_Eco_20-4

In order to evaluate the bacteriolytic activity of the bacteriophage CJ_Eco_20-4, Time-kill assay was performed. Specifically, after culturing the ETEC-11 strain separated from fecal samples collected from hog farms of Gyeonggi-do area of Korea at 30°C and 180 rpm for 3 hours, it was diluted so that the absorbance (O.D.) was 0.2 at 600 nm, and a cultured solution of 2 X 10 ⁸ CFU/mL was put in tubes by 30 mL each and 4 tubes were prepared each. The concentrated solution of the bacteriophage CJ_Eco_20-4 was prepared by 2 X 10 ⁹ PFU/mL, and was added to each tube in which the ETEC strain cultured solution was put so that MOI (multiplicity of infection) was 0.1, 1 or 10, and as a control group, an ETEC strain cultured solution in which the bacteriophage was added was used. While culturing each tube at 30°C and 180 rpm for 6 hours, 1 mL of cultured solution was collected at 1 hour intervals and the absorbance was measured at 600 nm.

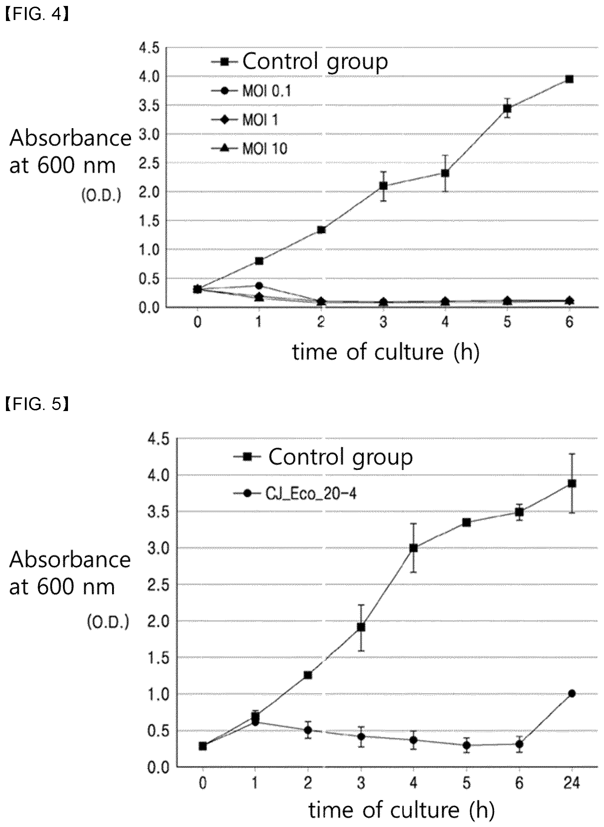

As a result, as shown in FIG. 4, in 6 hours after culturing, while the absorbance was increased by 4.0 in the control group, the absorbance was shown as 0.1 even in a group in which MOI was the lowest, 0.1, in the ETEC strain cultured solution in which the bacteriophage CJ_Eco_20-4 was added, and thus, it could be seen that the bacteriophage CJ_Eco_20-4 had very strong bacteriolytic activity.

In addition, as a result of proceeding an additional experiment in the same manner as described above except for measuring the absorbance of the culture solution by culturing the control group and the ETEC strain cultured solution to which the bacteriophage CJ_Eco_20-4 was added at MOI 0.1 for 24 hours in the above experiment, as shown in FIG. 5, it was confirmed that the bacteriophage CJ_Eco_20-4 showed strong bacteriolytic activity up to 24 hours even when it was treated at a low MOI of 0.1.

## Claims

1. Bacteriophage CJ_Eco_20-4 which has specific bactericidal activity against enterotoxigenic *Escherichia coli* (ETEC), and is deposited under Accession number KCCM12936P.

2. A composition for use in prevention or treatment of infectious disease caused by enterotoxigenic *Escherichia coli,*
wherein the composition comprises the bacteriophage CJ_Eco_20-4 of claim 1 as an active ingredient.

3. The composition for use according to claim 2, wherein the infectious disease caused by enterotoxigenic *Escherichia coli* is colibacillosis.

4. An antibiotic comprising the bacteriophage CJ_Eco_20-4 of claim 1 as an active ingredient.

5. A feed additive comprising the bacteriophage CJ_Eco_20-4 of claim 1 as an active ingredient.

6. A feed comprising the feed additive of claim 5.

7. A drinking water additive comprising the bacteriophage CJ_Eco_20-4 of claim 1 as an active ingredient.

8. Drinking water comprising the drinking water additive of claim 7.

9. A disinfectant comprising the bacteriophage CJ_Eco_20-4 of claim 1 as an active ingredient.

10. A detergent comprising the bacteriophage CJ_Eco_20-4 of claim 1 as an active ingredient.

11. The bacteriophage according to claim 1, for use in prevention or treatment of infectious disease caused by enterotoxigenic *Escherichia coli* (ETEC).

## Patentansprüche

1. Bakteriophage CJ_Eco_20-4, der eine spezifische bakterizide Wirkung gegen enterotoxigene *Escherichia coli* (ETEC) aufweist und unter der Zugangsnummer KCCM12936P hinterlegt ist.

2. Zusammensetzung zur Verwendung bei der Vorbeugung gegen oder Behandlung von Infektionskrankheiten, die durch enterotoxigene *Escherichia coli* verursacht werden,
wobei die Zusammensetzung den Bakteriophagen CJ_Eco_20-4 nach Anspruch 1 als Wirkstoff umfasst.

3. Zusammensetzung zur Verwendung gemäß Anspruch 2, wobei die durch enterotoxigene *Escherichia coli* verursachte Infektionskrankheit Colibazillose ist.

4. Antibiotikum, das den Bakteriophagen CJ_Eco_20-4 nach Anspruch 1 als Wirkstoff umfasst.

5. Futtermittelzusatz, der den Bakteriophagen CJ_Eco_20-4 nach Anspruch 1 als Wirkstoff umfasst.

6. Futtermittel, das den Futtermittelzusatz nach Anspruch 5 umfasst.

7. Trinkwasserzusatz, der den Bakteriophagen CJ_Eco_20-4 nach Anspruch 1 als Wirkstoff umfasst.

8. Trinkwasser, das den Trinkwasserzusatz nach Anspruch 7 umfasst.

9. Desinfektionsmittel, das den Bakteriophagen CJ_Eco_20-4 nach Anspruch 1 als Wirkstoff umfasst.

10. Reinigungsmittel, das den Bakteriophagen CJ_Eco_20-4 nach Anspruch 1 als Wirkstoff umfasst.

11. Bakteriophage nach Anspruch 1 zur Verwendung bei der Vorbeugung gegen oder Behandlung von Infektionskrankheiten, die durch enterotoxigene *Escherichia coli* (ETEC) verursacht werden.

## Revendications

1. Bactériophage CJ_Eco_20-4 qui possède une activité bactéricide spécifique contre *Escherichia coli* entérotoxinogène (ETEC), et qui est déposé sous le numéro de dépôt KCCM12936P.

2. Composition pour utilisation dans la prévention ou le traitement d'une maladie infectieuse causée par *Escherichia coli* entérotoxinogène, où la composition comprend le bactériophage CJ_Eco_20-4 selon la revendication 1 en tant que principe actif.

3. Composition pour utilisation selon la revendication 2, où la maladie infectieuse causée par *Escherichia coli* entérotoxinogène est la colibacillose.

4. Antibiotique comprenant le bactériophage CJ_Eco_20-4 selon la revendication 1 en tant que principe actif.

5. Additif alimentaire comprenant le bactériophage CJ_Eco_20-4 selon la revendication 1 en tant que principe actif.

6. Aliment comprenant l'additif alimentaire selon la revendication 5.

7. Additif pour eau potable comprenant le bactériophage CJ_Eco_20-4 selon la revendication 1 en tant que principe actif.

8. Eau potable comprenant l'additif pour eau potable selon la revendication 7.

9. Désinfectant comprenant le bactériophage CJ_Eco_20-4 selon la revendication 1 en tant que principe actif.

10. Détergent comprenant le bactériophage CJ_Eco_20-4 selon la revendication 1 en tant que principe actif.

11. Bactériophage selon la revendication 1, pour utilisation dans la prévention ou le traitement d'une maladie infectieuse causée par *Escherichia coli* entérotoxinogène (ETEC).
